# EUROPÄISCHE PATENTSCHRIFT

(11) **EP 1 435 253 B1**
(45) Veröffentlichungstag und Bekanntmachung des Hinweises auf die Patenterteilung: **17.01.2007**
(21) Anmeldenummer: 02029115.9
(22) Anmeldetag: 31.12.2002
(51) Int. Cl.: A61M 25/00, A61M 25/10

(54) **Katheter mit einem flexibleren Bereich zwischen Schaft und Spitze sowie Verfahren zu seiner Herstellung**
Catheter having a more flexible part between shaft and tip and method of manufacturing thereof
Cathéter avec partie plus flexible entre la tige et la pointe distale et procédé de fabrication

(43) Veröffentlichungstag der Anmeldung: 07.07.2004
(73) Patentinhaber: Abbott Laboratories Vascular Enterprises Limited, Dublin 2 (IE)
(72) Erfinder: Quint, Bodo, 72108 Rottenburg-Seebronn (DE)
(74) Vertreter: Weber, Joachim

(56) Entgegenhaltungen:
- US-A- 3 938 502
- US-A- 4 782 834
- US-A- 5 171 232
- US-A- 5 242 394
- US-A- 5 554 121
- US-A- 5 746 701
- US-A- 5 971 975
- US-A1- 2002 029 031

## Beschreibung

Die Erfindung betrifft ein Verfahren zur Herstellung eines Katheters gemäß Anspruch 1.

An Verengungsstellen in Körpergefäßen oder Körperhöhlungen werden heutzutage zur Aufweitung der Verengung Katheteranordnungen verwendet, welche an ihrem distalen Ende einen Ballon aufweisen, der expandierbar ist. Auf diesen expandierbaren Ballon kann zusätzlich ein Stent aufgekrimpt sein, der zur Stabilisierung der Gefäßwand in der Verengung platziert werden kann. Die Katheteranordnung wird mit Hilfe eines Führungsdrahtes an die verengte Stelle im Körper des Patienten geführt und die Verengung durch Expansion des Ballons aufgeweitet bzw. der aufgekrimpte Stent in der Verengung platziert.

Aus dem US-Patent 4,782,834 ist ein Katheter mit einer weichen Spitze bekannt, jedoch wird bei diesem Katheter die weiche Spitze durch einen Materialübergang auf ein Material geringerer Festigkeit erreicht. Dies bedeutet, dass die gesamte Spitze aus dem weichen Material besteht, was das Problem aufwirft, dass dieser Bereich zum Ausknicken neigt. Mögliche Folge ist eine Deformation der Spitze, die zur Verengung des Innenlumens oder bleibender Deformation führen kann.

Aus der US-A 5,971,975 ist ein weiterer Katheter mit einem Katheterschaft und einer Katheterspitze bekannt, wobei der Katheterschaft einen Bereich aufweist, der aus einem Material besteht, das biegeweicher ist als das Material des Katheterschafts und das proximal der Katheterspitze angeordnet ist.

Aus der US-A 5,504,121 ist ein Katheter bekannt, der einen Katheterschaft mit einem äußeren rohrförmigen Teil aufweist, das ein Gelenk aufweist, das beispielsweise durch Wärmeaufbringung oder Laserfusion herstellbar ist.

Weitere Katheterkonstruktionen sind aus der US-A 5,242,394, der US 3,938,502 und der EP-A2-0 334 640 bekannt.

Die Qualität eines Ballonkatheters zeichnet sich dadurch aus, dass der Katheter möglichst einfach gewundenen Gefäßabschnitten folgen kann und der Katheter möglichst weit, gelenkt durch den Führungsdraht ("guide wire"), in eine Stenose vorgeschoben werden kann ("Tracking"). Dieses "Tracking" wird dadurch unterstützt, dass die Spitze flexibel genug ist, um den Balloon zu lenken. Wird daher bevorzugt die distale Spitze - i.a. "Tip" genannt - aus einem hierfür besonders geeigneten biegeweicheren Material gestaltet, folgt das gesamte System den Windungen des Gefäßes. Gleichzeitig muss jedoch gewährleistet sein, dass der Katheter in die verengten Bereiche des Gefäßes sicher vorgeschoben werden kann. Diese Eigenschaft wird durch den Fachbegriff "Pushability" beschrieben. Falls die gesamte Katheterspitze, wie bei derjenigen des US-Patentes 4,782,834 aus einem weichen Material gefertigt wird, wird gerade diese geforderte Eigenschaft negativ beeinflusst. Unter "Push" kann es zu reversibler oder irreversibler Deformation der Katheterspitze kommen und damit auch zur Verhakung am "guide wire" (Friction). Je kleiner das Profil der Spitze ist, d.h. je kleiner das sogenannte "leasion entry profile" oder Eingangsprofil ist, desto mechanisch empfindlicher ist die Spitze und desto ungünstiger wirkt sich weiches Material an der Spitze auf ihre Eigenschaften aus.

Da zum Durchdringen enger Stenosen jedoch ein möglichst kleines Eingangsprofil des Katheters erwünscht ist, wäre es daher ideal, eine Spitze formen zu können, welche ein minimales Eingangsprofil aufweist, aber mechanisch stabil ist, einen kontinuierlich zunehmenden Durchmesser aufweist, aber flexibel ist und homogen in den Ballonkonus übergeht. Die vorliegende Erfindung stellt einen Katheter und ein Verfahren zu dessen Herstellung dar, welcher diese mechanischen Eigenschaften der Spitze zur Verfügung stellt.

Es ist Aufgabe der vorliegenden Erfindung, ein Verfahren zur Herstellung eines Katheters zu schaffen, mit dem es möglich ist, ein in alle Richtungen bewegliches Gelenk im Katheter proximal der Katheterspitze zu schaffen.

Die Lösung dieser Aufgabe erfolgt durch die Merkmale des Anspruches 1.

Mit dem erfindungsgemäßen Verfahren ist es möglich, einen Katheter herzustellen, dessen Katheterspitze ein Gelenk aufweist, das aus einem Material besteht, das biegeweicher ist, als das Material des Katheterschaftes. Dieses Gelenk wird bei Ballonkathetern zwischen dem distalen Ende des Ballons und dem distalen Ende der Katheterspitze angeordnet. Hierbei ist es möglich, dass das distale Ende der Katheterspitze aus demselben Material wie der Katheterschaft besteht. Bevorzugterweise besteht das distale Ende der Katheterspitze aus einem festeren Material als der Katheterschaft. Die Katheterspitze kann beispielsweise eine Metallspitze, ein Metallring oder aus PTFE bestehen, sie kann beschichtet, galvanisiert oder röntgensichtbar sein.

Diese Anordnung gewährleistet eine nahezu optimale Kombination zwischen biegeweicher Spitze und hoher Pushability.

Die Unteransprüche haben vorteilhafte Weiterbildungen der Erfindung zum Inhalt.

Im Einzelnen wird erfindungsgemäß ein Verfahren zur Herstellung des zuvor erläuterten Katheters angegeben, bei dem zunächst auf den Bereich zwischen dem distalen Ende des Ballons und dem Vorderende des Katheterschaftes ein Material, beispielsweise in Form eines Materialringes, aufgebracht wird, das biegeweicher ist als dasjenige des Katheterschaftes. Zur Vorfixierung kann dieser Materialring durch einen Schrumpfschlauch auf dem Katheterschaft befestigt werden. Danach erfolgt eine Verschweißung durch Erhitzen mit geeigneter Strahlungsenergie, beispielsweise monochromatischem oder polychromatischem Licht, Laserlicht, elektromagnetischer Strahlung, Heißluft oder Hitze, was das biegeweichere Material an der gewünschten Stelle durch den erforderlichen Materialfluss bzw. die Materialverdrängung in den Katheterschaft integriert. Die Schweissung erfolgt dabei vorzugsweise rotationssymmetrisch durch schnelle, agressive Energieeinwirkung. Nach dieser Integrierung wird der Schrumpfschlauch entfernt und das in alle Richtungen bewegliche Gelenk ist an der gewünschten Stelle im Katheterschaft in dessen distalem Spitzenbereich angebracht.

Weitere Einzelheiten, Merkmale und Vorteile der Erfindung ergeben sich aus nachfolgender Beschreibung von Ausführungsbeispielen und anhand der Zeichnungen.

Es zeigt:
- Fig. 1: eine schematisch vereinfachte Darstellung eines erfindungsgemäßen Katheters,
- Fig. 2: eine der Fig. 1 entsprechende Darstellung des distalen Endbereiches des Katheters gemäß Fig. 1 zur Erläuterung des erfindungsgemäßen Verfahrens, und
- Fig. 3: eine der Fig. 1 entsprechende Darstellung einer zweiten Ausführungsform des erfindungsgemäßen Katheters.

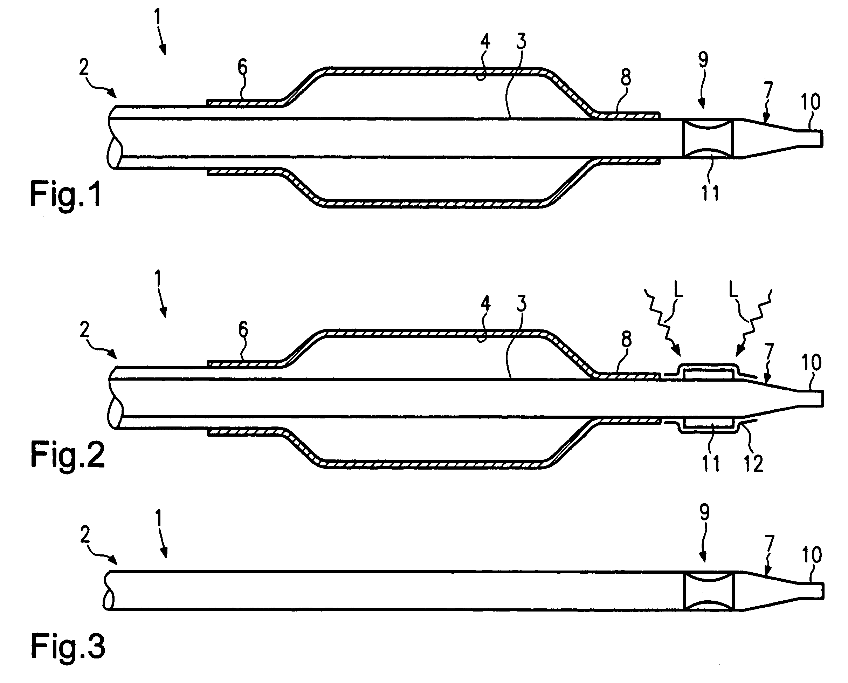

Der erfindungsgemäße in Fig. 1 dargestellte Katheter 1 weist einen in üblicher Art und Weise ausgebildeten Katheterschaft 2 auf, an dessen distalem Ende 3 ein Ballon 4 mit einem proximalen Ende 6 und einem distalen Ende 8 angeordnet ist.

Der Katheterschaft 2 weist eine Katheterspitze 7 auf, die ein Teil des Katheterschaftes 2 ist, und die über das distale Ende 8 des Ballons 3 vorragt.

Die Katheterspitze 7 des erfindungsgemäßen Katheters 1 weist ein Gelenk 9 auf, das aus einem Material besteht, das biegeweicher ist als das Material des Katheterschaftes 2. Dieses Gelenk 9 ist zwischen dem distalen Ende 8 des Ballons 4 und dem Vorderende 10 der Katheterspitze 7 bzw. des Katheterschaftes 2 angeordnet. Hierdurch wird einerseits eine biegeweiche Eigenschaft der Katheterspitze 7 erreicht, andererseits jedoch eine hohe Pushability gewährleistet, da das Vorderende 10 der Katheterspitze 7 aus demselben biegesteifen Material des Katheterschaftes 2 oder einem festeren Material besteht.

In Fig. 2 ist der distale Endbereich des Katheters 1 zur Erläuterung des erfindungsgemäßen Verfahrens dargestellt. Bei diesem Verfahren wird zunächst ein Material auf den Bereich zwischen dem distalen Ende 8 des Ballons und dem Vorderende 10 des Katheterschaftes 2 aufgebracht, das biegeweicher ist als das Material des Katheterschaftes 2. Dieses Material kann beispielsweise in Form eines Ringes 11 auf die gewünschte Stelle aufgebracht werden. Bei der in Fig. 2 dargestellten besonders bevorzugten Ausführungsform erfolgt eine Vorfixierung dieses Ringes 11 durch das Aufbringen einer Schrumpffolie 12. Danach wird Strahlungsenergie, die in Fig. 2 durch die beiden gewellten Pfeile L dargestellt ist, auf das Material 11 aufgebracht, so dass sich die in Fig. 1 dargestellte Integrierung dieses Materials 11 in das Material des Katheterschaftes 2 und damit die Bildung des biegeweichen Gelenkes 9 ergibt.

In Fig. 3 ist eine zweite Ausführungsform eines erfindungsgemäßen Katheters 1 dargestellt, der wiederum einen Katheterschaft 2 aufweist, der eine Katheterspitze 7 mit einem distalen Vorderende 10 aufweist.

Der in Fig. 3 dargestellte Katheter 1 weist jedoch keinen Ballon auf. Dementsprechend ist das Gelenk 9, das im Prinzip demjenigen der Fig. 1 und 2 entspricht, proximal der Katheterspitze 7 angeordnet.

Die Katheterspitze 7 kann wiederum aus dem gleichen Material wie der Katheterschaft 2 oder auch aus einem festeren Material bestehen.

Bei einer weiteren alternativen Ausführungsform kann am distalen Ende des Katheterschafts 2 ein in den Figuren nicht näher dargestellter Ultraschallkopf vorgesehen sein. Bei einer derartigen Ausführungsform ist das Gelenk 9 zwischen diesem Ultraschallkopf und dem distalen Ende 10 der Katheterspitze 7 angeordnet.

## Patentansprüche

1. Verfahren zur Herstellung eines Katheters (1) mit einem Katheterschaft (2), und einer Katheterspitze (7), die ein Teil des Katheterschaftes (2) ist, **gekennzeichnet durch** folgende Verfahrensschritte:
- Aufbringen eines Materials (11) auf die Katheterspitze (7), das weicher ist als das Material des Katheterschaftes (2);
- Bestrahlen des Materials (11) mit Strahlungsenergie (L);
- Verschweißen des Materials (11) mit dem Material des Katheterschaftes zur Bildung eines Gelenks (9), wobei durch Materialverdrängung das weichere Material (11) in das Material des Katheterschaftes (2) integriert wird.

2. Verfahren nach Anspruch 1, **dadurch gekennzeichnet, dass** das Material (11) vor der Verschweißung vorfixiert wird.

3. Verfahren nach Anspruch 1 oder 2, **dadurch gekennzeichnet, dass** zur Vorfixierung ein Schrumpfschlauch (12) verwendet wird.

4. Katheter (1)
- mit einem Katheterschaft (2),
- mit einer Katheterspitze (7), und
- mit einem Gelenk (9) zwischen dem Katheterschaft (2) und der Katheterspitze (7),
**dadurch gekennzeichnet,**
- **dass** das Gelenk (9) von einem durch Materialverdrängung in das Material des Katheterschaftes (2) integrierten weicheren Material als demjenigen des Katheterschaftes (2) gebildet ist.

## Claims

1. A method for manufacturing a catheter (1) comprising a catheter shaft (2) and a catheter tip (7) being part of the catheter shaft (2), **characterized by** the following method steps:
- applying a material (11) onto the catheter tip (7), said material being softer than the material of the catheter shaft (2);
- treating the material (11) with a radiation energy (L);
- welding the material (11) with the material of the catheter shaft to form a joint (9), wherein the softer material (11) is integrated into the material of the catheter shaft (2) by way of material crowding.

2. The method of claim 1, **characterized in that** the material (11) is pre-fixated prior to the welding step.

3. The method of claim 1 or 2, **characterized in that** a shrinkdown tubing (12) is used for the pre-fixation.

4. A catheter (1), comprising
- a catheter shaft (2),
- a catheter tip (7), and
- a joint (9) between the catheter shaft (2) and the catheter tip (7),
**characterized in that**
- the joint (9) is formed by a material integrated into the material of the catheter shaft (2) by way of material crowding, said material being softer than that of the catheter shaft.

## Revendications

1. Procédé de réalisation d'un cathéter (1), comportant une tige (2) et une pointe (7) qui constitue une partie de la tige (2), **caractérisé par** les étapes de procédé suivantes :
- application d'un matériau (11) sur la pointe (7) du cathéter, lequel est plus mou que le matériau de la tige (2) du cathéter ;
- exposition du matériau (11) à une énergie de rayonnement (L) ;
- soudage du matériau (11) avec le matériau de la tige du cathéter pour former une articulation (9), le matériau (11) plus mou étant intégré dans le matériau de la tige (2) du cathéter sous l'effet d'un refoulement de la matière.

2. Procédé selon la revendication 1, **caractérisé en ce que** le matériau (11) est préfixé avant le soudage.

3. Procédé selon la revendication 1 ou 2, **caractérisé en ce qu'**un tuyau flexible thermorétractable (12) est utilisé pour la pré-fixation.

4. Cathéter (1)
- comportant une tige (2),
- comportant une pointe (7), et
- comportant une articulation (9) entre la tige (2) et la pointe (7),
**caractérisé en ce que**
- l'articulation (9) est formée par un matériau plus mou que celui de la tige (2) du cathéter, lequel est intégré dans le matériau de la tige (2) du cathéter sous l'effet d'un refoulement de la matière.
